# EUROPEAN PATENT SPECIFICATION

(11) **EP 0 763 360 B1**
(45) Date of publication and mention of the grant of the patent: **16.02.2005**
(21) Application number: 95914555.8
(22) Date of filing: 10.04.1995
(51) Int. Cl.: A61K 38/36, A61K 38/18

(54) **USE OF THROMBOMODULIN FOR TREATING LIVER INJURY**
VERWENDUNG VON THROMBOMODULIN GEGEN LEBERERKRANKUNGEN
UTILISATION DE LA THROMBOMODULINE CONTRE L'HEPATOPATHIE

(30) Priority: 20.04.1994 JP 8119694
(43) Date of publication of application: 19.03.1997
(73) Proprietor: Asahi Kasei Pharma Corporation, Tokyo 101-8481 (JP)
(72) Inventor: FUJIWARA, Kenji, Oomiya-shi, Saitama-ken 331 (JP); MOCHIDA, Satoshi, Tokyo 179 (JP)
(74) Representative: Boeters, Hans Dietrich, Dr.
(86) International application number: PCT/JP1995/000704
(87) International publication number: WO 1995/028953

(56) References cited:
- EP-B- 0 312 598
- JP-A- 5 310 787
- CHEMICAL ABSTRACTS, Vol. 119, (1993), Abstract Number 92796r (K. MORI et al.). XP002956960
- CHEMICAL ABSTRACTS, Vol. 119, (1993), Abstract Number 46674b (K. AKIYAMA et al.). XP002956961
- CHEMICAL ABSTRACTS, Vol. 115, (1991), Abstract Number 6273f (C. SHIMAZU et al.). XP002956962
- CHEMICAL ABSTRACTS, Vol. 121, (1994), Abstract Number 154668c (O. CHAZOUILLERES et al.). XP002956963

## Description

### BACKGROUND OF THE INVENTION

### Field of the Invention

The present invention relates to a composition for treating liver injury. More particularly, the present invention is concerned with use of thrombomodulin for producing a composition for treating liver injury, wherein the composition comprises a thrombomodulin as an active ingredient and an at least one pharmaceutically acceptable carrier. The composition of the present invention very effectively acts on liver injury, such as fulminant hepatitis, to remarkably ameliorate the injury.

### Prior Art

Liver is the largest single organ in a living body, and is the principal site of saccharometabolism, protein metabolism and lipid metabolism. In addition, drug metabolism, detoxification, storage of substances, and synthesis of proteins, such as a protein participating in blood coagulation, are also important functions of the liver. Thus, the liver is an essential organ to the maintenance of homeostasis and life. In a physiological view, the above-mentioned functions are the result of individual actions or interactions of the cells which constitute the liver, such as liver parenchyma cells, Kupffer cells and sinusoid endothelial cells. As mentioned above, the liver is an essential organ to a living body. Therefore, once a living body suffers liver injury due to the invasion of a virus from the exterior, the intake of alcohol, or the like, the body is likely to fall into a serious condition.

With respect to liver injury, various causes can be mentioned, for example, toxic substances which directly damage liver parenchyma cells and the metabolism thereof; active oxygen and peroxylipids which are generated during the metabolism of a substance, such as an alcohol; microcirculation injury resulting from a local progress of blood coagulation; and an autoimmunity which is induced by the infection with a hepatitis virus, such as hepatitis B virus.

A number of types of compositions for treating liver injury ascribed to the above-mentioned various causes have been used. However, no therapeutically definitive composition for treating liver injury has yet been developed. This fact is described in detail in the publication, "The Journal of Practical Pharmacy, 41(11) (1990)". For example, this publication describes that the majority of parenteral fluids containing amino acids in specific formulations and of vitamin supplements serve only as an auxiliary treatment, such as treatment for supplementing the shortage of essential nutrients and vitamins which is caused by the depression of the metabolisms in liver, and also describes that, with respect to antidotes, representative examples of which include SH compounds, such as glutathione and thiopronine, their detoxification effects are uncertain. Further, according to this publication, various drugs having therapeutic effects against liver injury, such as anti-inflammatory or immunomodulating drugs (e.g., an adrenal cortical hormone, glycylrhizin, azathioprine and the like); protein synthesis accelerators (e.g., malothilate and the like); and interferons having antiviral activity, are known to cause serious side effects, such as hepatic disorder, hypoaldosteronism, agranulocytosis, icterus and cardiomyopathy.

Incidentally, a thrombomodulin is a substance which has the ability to specifically bind to thrombin to inhibit blood coagulation activity of the thrombin, and to promote the activation of protein C by thrombin. Thus, a thrombomodulin is known to have a strong anti-blood coagulation activity. By animal experiments, it has been demonstrated that the thrombomodulin is effective for treatment and prevention of diseases which accompany the progress of blood coagulation, such as thrombosis and disseminated intravascular coagulation (DIC) [see K. Gomi et. al., Blood, 75, 1396-1399 (1990)].

The thrombomodulin is a glycoprotein which is present on the membrane of vascular endothelial cells. Recently, a thrombomodulin is produced by genetic engineering techniques. The cloning of human thrombomodulin cDNA has been reported (see S. Yamamoto et. al, International Application Publication No. WO88/05053), and it has been elucidated that the human thrombomodulin is a membrane-bound protein composed of domain 1 (N-terminal domain), domain 2 (EGF domains), and domain 3 (O-glycosylation site rich domain), wherein domains 1 to 3 are extracellular domains, domain 4 (transmembrane domain) is a hydrophobic region, and domain 5 is an intracellular domain [see The EMBO Journal, 6(7), 1891-1897 (1987)], wherein domains 1 to 5 are arranged in that order starting from the N-terminus thereof. Further, it is known that the minimum active unit of the thrombomodulin is present in the 4th to 6th EGF domains among the six EGF domains constituting domain 2, and that a peptide fragment comprising these 4th to 6th EGF domains of the thrombomodulin is also considered to be effective for the treatment of diseases which accompany the progress of blood coagulation, such as thrombosis and DIC [see M. Zushi et. al., J. Biol. Chem., 266, 19886-19889 (1991)].

However, no reports have been found with respect to the pharmacological effects of a thrombomodulin on diseases other than diseases which accompany the progress of blood coagulation, such as thrombosis and DIC.

### SUMMARY OF THE INVENTION

The present inventors have made extensive and intensive studies with a view toward developing a medicine effective for treating liver injury. As a result, it has surprisingly been found that a thrombomodulin, which is known as having the ability to inhibit blood coagulation, is also effective for treating liver injury. Illustratively stated, the present inventors have unexpectedly found that, when a thrombomodulin was experimentally administered to a liver injury model which had been prepared by excising a part of liver from a rat and administering lipopolysaccharide (LPS) to the rat having its liver partly excised, a remarkable improvement was observed in the suppression of the amount of enzymes liberated to serum from the liver, wherein the amount of liberated enzymes is a criterion for liver injury. Further, when the liver tissue of the above-mentioned liver injury model of a rat administered with a thrombomodulin was observed under a light microscope, it was found that the liver injury was remarkably ameliorated. Furthermore, it was found that, when a thrombomodulin was experimentally administered to a mouse liver injury model, which had been prepared by administration with thermally killed microorganisms and LPS, a significant improvement in survival ratio was observed. The above-mentioned liver injury model administered with LPS, or thermally killed microorganisms and LPS, has been regarded as a model which is capable of reproducing a clinical picture of liver injury [see Bioclinica, 9(4), 238-242 (1994) and Nihon Syokaki Gakkai Zasshi (The Japanese Journal of Gostroenterological Surgery), 83(6), 1161-1167 (1986)]. The present invention has been completed based on the above findings.

Therefore, it is an object the present invention to provide use of thrombomodulin for producing a composition for treating liver injury, wherein the composition comprises a thrombomodulin as an active ingredient and at least one pharmaceutically acceptable carrier, and effectively acts on liver injury to remarkably ameliorate the liver injury.

The foregoing and other objects, features and advantages of the present invention will be apparent to those skilled in the art from the following detailed description and appended claims taken in connection with the accompanying sequence listings and drawings.

### BRIEF DESCRIPTION OF THE SEQUENCE LISTINGS

In the sequence listings:
SEQ ID NO. 1 is the amino acid sequence which corresponds to the 349th to 462nd amino acids of the entire amino acid sequence of natural human thrombomodulin; and
SEQ ID NO. 2 is the amino acid sequence which corresponds to the 1st to 498th amino acids of the entire amino acid sequence of natural human thrombomodulin.

### BRIEF DESCRIPTION OF THE DRAWINGS

In the drawings:
Fig. 1 is a photomicrograph of liver tissue of a rat liver injury model, which was administered with physiological saline in Example 1; and
Fig. 2 is a photomicrograph of liver tissue of a rat liver injury model, which was administered with thrombomodulin in Example 1.

### DETAILED DESCRIPTION OF THE INVENTION

In one aspect of the present invention, there is provided a composition for treating liver injury, which comprises an effective anti-liver injury amount of a thrombomodulin and at least one pharmaceutically acceptable carrier.

A thrombomodulin which is an active ingredient of the composition of the present invention is a substance having the ability to bind to thrombin and to promote the activation of protein C by thrombin. Examples of thrombomodulins include human thrombomodulin and a peptide fragment thereof. Preferred are peptides respectively comprising amino acid sequences of SEQ ID NOs. 1 and 2. Especially preferred are a thrombomodulin consisting of an amino acid sequence of SEQ ID NO. 1 and a thrombomodulin consisting of an amino acid sequence of SEQ ID NO. 2.

A thrombomodulin which lacks domain 4 as the transmembrane domain (hydrophobic domain), such as the above-mentioned thrombomodulin consisting of the amino acid sequence of SEQ ID NO. 1 or 2, is soluble in an aqueous medium without a need for being treated with a surfactant or the like. Therefore, the above-mentioned thrombomodulins respectively having the amino acid sequences of SEQ ID NOs. 1 and 2 can be advantageously used in preparing and using the composition of the present invention, since these thrombomodulins are easy to handle in preparation and use of the composition.

In the present invention, the term "soluble" means that the thrombomodulin can be dissolved in an aqueous medium in a concentration at which a promotion of the thrombin-catalyzed activation of protein C is detected, without a need for the thrombomodulin to be treated with a surfactant. For example, the term "soluble" means that the thrombomodulin can be dissolved, without a need for being treated with a surfactant, in a 20 mM Tris-HCl buffer (pH 7.4) containing 0.1 M sodium chloride, 2.5 mM calcium chloride and 1 mg/ml serum albumin (wherein the buffer has thrombin and protein C added thereto) in a concentration at which a significant promotion of the thrombin-catalyzed activation of protein C is detected (for example, in a concentration of 10 ng/ml or higher). In the present invention, a thrombomodulin having an amino acid sequence other than those of SEQ ID NOs. 1 and 2 can also be preferably used, as long as the thrombomodulin is soluble in an aqueous medium (that is, the thrombomodulin has no transmembrane domain) and has the ability to promote the thrombin-catalyzed activation of protein C.

Also, by treating with a surfactant, natural human thrombomodulin having the entire amino acid sequence of 557 amino acids, containing a transmembrane domain as a hydrophobic domain and an intracellular domain in addition to the extracellular domain, can be rendered usable as the "thrombomodulin" in the present invention.

The thrombomodulin used in the present invention may contain a sugar residue, such as chondroitin sulfate.

A thrombomodulin can be produced according to a customary method, and reference can be made, for example, to International Application Publication No. WO 88/05053 (S. Yamamoto et al.), J. Biol. Chem., 266, 19886-19889 (1991) (M. Zushi et al.), and J. Biol. Chem., 257, 859-864 (1982) (C.T. Esmon et al.).

In the present invention, the term "liver injury" means not only degeneration or necrosis of liver parenchyma cells which results from injury caused by a certain factor, but also undesirable phenomena caused by biological reactions to the injury, such as mobilization, infiltration, activation of Kupffer cells, leukocytes and the like, fibrosis of the liver tissue, etc., which reactions occur alone or in combination. A "liver injury" can be classified into one of four types, i.e., acute, subacute, chronic and fulminant types, depending on the development of symptoms. Especially noted is fulminant hepatitis, which is one type of liver injury exhibiting symptoms of liver failure (such as disturbance of consciousness) caused by extensive necrosis of liver cells during the course of acute hepatitis.

The composition of the present invention for treating liver injury can be obtained as a mixture of an effective anti-liver injury amount of a thrombomodulin with at least one pharmaceutically acceptable carrier. That is, the composition which is suitable for administration to a patient can be prepared by mixing an effective anti-liver injury amount of a thrombomodulin with an appropriate amount of at least one conventional carrier. For example, when the composition of the present invention is used in the form of an injection, there may be employed as an additive, such as sucrose, glycerol, a pH adjusting agent of various inorganic salts.

The composition of the present invention can be administered by a conventional method used for peptide drugs, i.e., parenteral administration, such as intravenous, intramuscular or subcutaneous administration. Alternatively, oral, rectal, nasal or sublingual administration can also be employed.

The dose of the composition of the present invention varies depending on the age, weight, conditions, etc. of the patient and the manner of administration. In general, however, the dose is about 0.001 to about 20 mg per kg of a patient. The present composition may be administered once a day or, if desired, several times a day.

### BEST MODE FOR CARRYING OUT THE INVENTION

Hereinbelow, the present invention will be described in more detail with reference to the following Examples and Comparative Example, but they should not be construed as limiting the scope of the present invention.

### Example 1

From each of a predetermined number of SD rats (male), about 70 % of the liver was excised. 48 Hours after the excision, 200 µg/kg of lipopolysaccharide (LPS) (which had been prepared from E. coli 026 : B6 strain manufactured and sold by Difco Laboratories, U.S.A.) and 0.4 ml of a test solution as described below were simultaneously administered to each of the rats through a caudal vein thereof. The above procedure was conducted in accordance with the method of Mochida et al. [Gastroenterology, 99, 771 - 777 (1990)]. The test solution had been prepared as follows: a thrombomodulin having the amino acid sequence of SEQ ID NO. 2 (hereinafter, frequently referred to simply as "TMD123") was produced in accordance with the method as described in Example 10 and Example 20 of International Patent Application Publication No. WO88/05053 (S. Yamamoto et al.), and two different amounts of the produced TMD123 were individually dissolved in 0.4 ml of physiological saline to prepare two different test solutions, wherein the two different test solutions respectively have concentrations such that, when the two different test solutions were individually administered to SD rats, the amounts of the administered TMD123 became 1 mg and 3 mg per kg of SD rat, respectively.

5 Hours after the administration, a blood sample was taken out from the femoral vein of the rat, and the GPT (glutamic pyruvic transaminase) value of the serum of the blood sample was determined in accordance with the method described in "Rinshou Kensahou Teiyou (Manual of Clinical Testing), 30th edition, p. 656, 1993 (published by Kanehara & Co., Ltd., Japan)".

On the other hand, as controls, male SD rats were individually administered with physiological saline instead of the above test solution, and the GPT values of the respective sera of the rats were determined by the same method as mentioned above.

Results are shown in Table 1.

**Table 1**

| | GPT value (kU*) average ± standard deviation (SD) |
|---|---|
| Rats administered with physiological saline (Number of rats: 11) | 1299 ± 1723 |
| Rats administered with 1 mg/kg of TMD123 (Number of rats: 7) | 244 ± 89 |
| Rats administered with 3 mg/kg of TMD123 (Number of rats: 7) | 184 ± 52 |

| | |
|---|---|
| *: kilounit | |

As shown in Table 1, the rats administered with TMD123 had an extremely low GPT value as compared to the rats administered with physiological saline. [As described in Comparative Example 1 below, the serum from the rats administered with heparin (which is known as a substance having the ability to inhibit blood coagulation) showed no significant difference in GPT value from the serum from the rats administered with physiological saline.]

With respect to each of the rats administered with 3 mg/kg of TMD123 and the rats administered with physiological saline, the liver tissue was dyed with hematoxylin and eosin, and the dyed tissue was observed under light microscope (120x magnification).

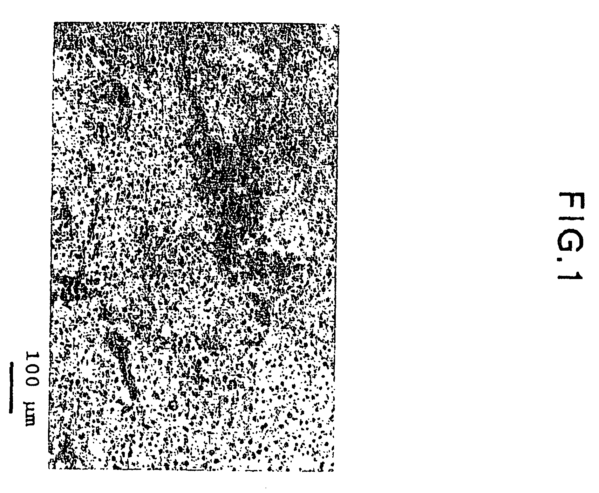

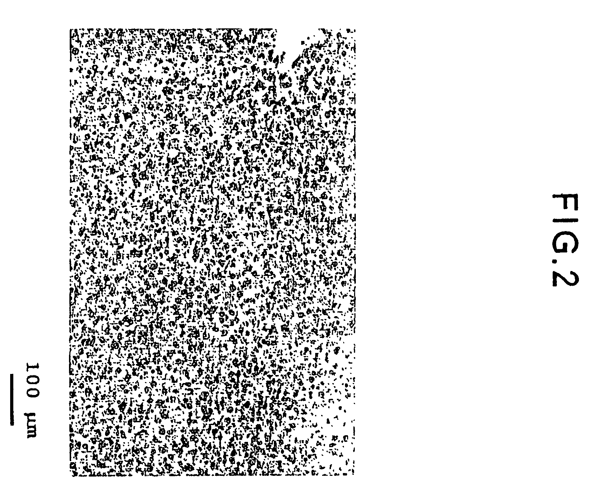

The photomicrograph of the liver tissue of the rat administered with physiological saline is shown in Fig. 1, and the photomicrograph of the liver tissue of the rat administered with TMD123 is shown in Fig. 2.

As is seen from Figs. 1 and 2, with respect to the liver tissue of the rat administered with physiological saline, extensive necrosis of liver cells occurred (Fig. 1), whereas, with respect to the liver tissue of the rat administered with TMD123, no significant difference was observed from normal liver tissue, that is, necrosis of liver cells did not occur (Fig. 2).

From the above results, it can be seen that TMD123 is very useful for treating liver injury.

### Example 2

Substantially the same procedure as in Example 1 was repeated, except that, in preparing the test solution, a thrombomodulin having the amino acid sequence of SEQ ID NO. 1 (hereinafter, frequently referred to simply as "TME456"), which is described in M. Zushi et al., J. Biol. Chem., 266, 19866 - 19889 (1991), was used instead of TMD123.

Results are shown in Table 2.

**Table 2**

| | GPT value (kU*) average ± standard deviation (SD) |
|---|---|
| Rats administered with physiological saline (Number of rats: 11) | 1255 ± 1682 |
| Rats administered with 1 mg/kg of TME456 (Number of rats: 7) | 296 ± 95 |
| Rats administered with 3 mg/kg of TME456 (Number of rats: 7) | 216 ± 58 |

| | |
|---|---|
| *: kilounit | |

As shown in Table 2, the rats administered with TME456 had an extremely low GPT value as compared to the rats administered with physiological saline. Therefore, it can be seen that TME456 is useful for treating liver injury.

### Example 3

Lethal models of mice suffering from acute liver failure were obtained in accordance with a partially modified method of the method of Mizoguchi et al. [Journal "Enshou (Inflammation)", 10, 115-118 (1990)]. Illustratively stated, 1 mg (dry weight) of Propionibacterium acnes (ATCC 11827) which had been killed by heating was dissolved in 0.2 ml of physiological saline, and the resultant solution was administered to each of male ICR mice through a caudal vein thereof. 7 Days after the administration, a solution of 1 µg of LPS (prepared from E. coli 0127 : BB strain manufactured and sold by Difco Laboratories, U.S.A.) in 0.1 ml of physiological saline, and a test solution (which had been obtained by dissolving TMD123 in 0.1 ml of physiological saline and had a concentration such that, when the test solution was administered to each of ICR mice, the amount of administered TMD123 became 3 mg per kg of the mouse) were simultaneously administered to each of the male ICR mice through a caudal vein thereof, and a survival ratio was determined 6 hours and 24 hours after the above administration of the LPS solution and the test solution. On the other hand, as controls, male ICR mice, to which physiological saline was administered instead of the above test solution, were tested.

Results are shown in Table 3.

**Table 3**

| | Survival ratio (%) | |
|---|---|---|
| | 6 hours after the administration | 24 hours after the administration |
| Mice administered with physiological saline (Number of rats: 20) | 50.0 | 0 |
| Mice administered with TMD123 (Number of rats: 20) | 80.0 | 60.0 |

As shown in Table 3, the mice administered with TMD123 exhibited an extremely high survival ratio, as compared to the mice administered with physiological saline. From the above, it can be seen that TMD123 is very useful for treating liver injury.

### Example 4

Substantially the same procedure as in Example 3 was repeated, except that, in preparing a test solution, TME456 was used instead of TMD123.

Results are shown in Table 4.

**Table 4**

| | Survival ratio (%) | |
|---|---|---|
| | 6 hours after the administration | 24 hours after the administration |
| Mice administered with physiological saline (Number of rats: 20) | 50.0 | 0 |
| Mice administered with TME456 (Number of rats: 20) | 75.0 | 55.0 |

As shown in Table 4, the mice administered with TME456 exhibited an extremely high survival ratio, as compared to the mice administered with physiological saline. From the above, it can be seen that TME456 is very useful for treating liver injury.

### Example 5

Single dose toxicity tests were conducted, each with 5 rats, by administering TMD123 and TME456 individually to the rats (including male and female rats). As a result, it was found that none of the rats died, even when each of TMD123 and TME456 was individually administered in an amount of 180 mg/kg.

### Comparative Example 1

Substantially the same procedure as in Example 1 was repeated, except that, as a test solution, a solution of heparin (in the form of sodium salt of heparin, manufactured and sold by The Green Cross Corporation, Japan) in physiological saline (in which the concentration of heparin was adjusted so that, when the test solution was administered to each of the rats, the amount of the administered heparin became 150 U per kg of the rat) was used instead of a solution of TMD123. Heparin is known as a substance having the ability to inhibit blood coagulation.

Results are shown in Table 5.

**Table 5**

| | GPT value (kU*) average ± standard deviation (SD) |
|---|---|
| Rats administered with physiological saline (Number of rats: 7) | 924.3 ± 920.5 |
| Rats administered with heparin (Number of rats: 7) | 754.3 ± 875.7 |

| | |
|---|---|
| *: kilounit | |

As shown in Table 5, the serum from each of the rats administered with heparin showed no significant difference in GPT value from the serum from each of the rats administered with physiological saline.

### INDUSTRIAL APPLICABILITY

The composition of the present invention effectively acts on liver injury, such as fulminant hepatitis, to remarkably ameliorate the injury.

SEQUENCE LISTING

SEQ ID NO: 1

SEQUENCE LENGTH: 114

MOLECULE TYPE: Protein

SEQUENCE DESCRIPTION

Asp Pro Cys Phe Arg Ala Asn Cys Glu Tyr Gln Cys Gln Pro Leu Asn
1               5                   10                  15
Gln Thr Ser Tyr Leu Cys Val Cys Ala Glu Gly Phe Ala Pro Ile Pro
            20                  25                  30
His Glu Pro His Arg Cys Gln Met Phe Cys Asn Gln Thr Ala Cys Pro
        35                  40                  45
Ala Asp Cys Asp Pro Asn Thr Gln Ala Ser Cys Glu Cys Pro Glu Gly
    50                  55                  60
Tyr Ile Leu Asp Asp Gly Phe Ile Cys Thr Asp Ile Asp Glu Cys Glu
65                  70                  75                  80
Asn Gly Gly Phe Cys Ser Gly Val Cys His Asn Leu Pro Gly Thr Phe
                85                  90                  95
Glu Cys Ile Cys Gly Pro Asp Ser Ala Leu Val Arg His Ile Gly Thr
            100                 105                 110
Asp Cys

SEQ ID NO: 2

SEQUENCE LENGTH: 498

MOLECULE TYPE: Protein

SEQUENCE DESCRIPTION

Ala Pro Ala Glu Pro Gln Pro Gly Gly Ser Gln Cys Val Glu His Asp
1               5                   10                  15
Cys Phe Ala Leu Tyr Pro Gly Pro Ala Thr Phe Leu Asn Ala Ser Gln
            20                  25                  30
Ile Cys Asp Gly Leu Arg Gly His Leu Met Thr Val Arg Ser Ser Val
        35                  40                  45
Ala Ala Asp Val Ile Ser Leu Leu Leu Asn Gly Asp Gly Gly Val Gly
    50                  55                  60
Arg Arg Arg Leu Trp Ile Gly Leu Gln Leu Pro Pro Gly Cys Gly Asp
65                  70                  75                  80
Pro Lys Arg Leu Gly Pro Leu Arg Gly Phe Gln Trp Val Thr Gly Asp
                85                  90                  95
Asn Asn Thr Ser Tyr Ser Arg Trp Ala Arg Leu Asp Leu Asn Gly Ala
            100                 105                 110
Pro Leu Cys Gly Pro Leu Cys Val Ala Val Ser Ala Ala Glu Ala Thr
        115                 120                 125
Val Pro Ser Glu Pro Ile Trp Glu Glu Gln Gln Cys Glu Val Lys Ala
    130                 135                 140
Asp Gly Phe Leu Cys Glu Phe His Phe Pro Ala Thr Cys Arg Pro Leu
145                 150                 155                 160
Ala Val Glu Pro Gly Ala Ala Ala Ala Ala Val Ser Ile Thr Tyr Gly
                165                 170                 175
Thr Pro Phe Ala Ala Arg Gly Ala Asp Phe Gln Ala Leu Pro Val Gly
            180                 185                 190
Ser Ser Ala Ala Val Ala Pro Leu Gly Leu Gln Leu Met Cys Thr Ala

195                 200                 205

Pro Pro Gly Ala Val Gln Gly His Trp Ala Arg Glu Ala Pro Gly Ala

    210                 215                 220

Trp Asp Cys Ser Val Glu Asn Gly Gly Cys Glu His Ala Cys Asn Ala

225                 230                 235                 240

Ile Pro Gly Ala Pro Arg Cys Gln Cys Pro Ala Gly Ala Ala Leu Gln

                245                 250                 255

Ala Asp Gly Arg Ser Cys Thr Ala Ser Ala Thr Gln Ser Cys Asn Asp

            260                 265                 270

Leu Cys Glu His Phe Cys Val Pro Asn Pro Asp Gln Pro Gly Ser Tyr

        275                 280                 285

Ser Cys Met Cys Glu Thr Gly Tyr Arg Leu Ala Ala Asp Gln His Arg

    290                 295                 300

Cys Glu Asp Val Asp Asp Cys Ile Leu Glu Pro Ser Pro Cys Pro Gln

305                 310                 315                 320

Arg Cys Val Asn Thr Gln Gly Gly Phe Glu Cys His Cys Tyr Pro Asn

                325                 330                 335

Tyr Asp Leu Val Asp Gly Glu Cys Val Glu Pro Val Asp Pro Cys Phe

            340                 345                 350

Arg Ala Asn Cys Glu Tyr Gln Cys Gln Pro Leu Asn Gln Thr Ser Tyr

        355                 360                 365

Leu Cys Val Cys Ala Glu Gly Phe Ala Pro Ile Pro His Glu Pro His

    370                 375                 380

Arg Cys Gln Met Phe Cys Asn Gln Thr Ala Cys Pro Ala Asp Cys Asp

385                 390                 395                 400

Pro Asn Thr Gln Ala Ser Cys Glu Cys Pro Glu Gly Tyr Ile Leu Asp
                405                 410                 415
Asp Gly Phe Ile Cys Thr Asp Ile Asp Glu Cys Glu Asn Gly Gly Phe
            420                 425                 430
Cys Ser Gly Val Cys His Asn Leu Pro Gly Thr Phe Glu Cys Ile Cys
        435                 440                 445
Gly Pro Asp Ser Ala Leu Val Arg His Ile Gly Thr Asp Cys Asp Ser
    450                 455                 460
GLy Lys Val Asp Gly Gly Asp Ser Gly Ser Gly Glu Pro Pro Pro Ser
465                 470                 475                 480
Pro Thr Pro Gly Ser Thr Leu Thr Pro Pro Ala Val Gly Leu Val His
                485                 490                 495
Ser Gly

## Claims

1. Use of a thrombomodulin for producing a composition for treating liver injury, wherein said composition comprises an effective anti-liver injury amount of a thrombomodulin and at least one pharmaceutically acceptable carrier.

2. The use according to claim 1, wherein said thrombomodulin is a peptide comprising an amino acid sequence of SEQ ID NO. 1 or 2.

3. The use according to claim 1, wherein said thrombomodulin is a human thrombomodulin.

## Patentansprüche

1. Verwendung eines Thrombomodulins zur Herstellung einer Zusammensetzung für die Behandlung eines Leberschadens, wobei die Zusammensetzung eine gegen einen Leberschaden wirksame Menge eines Thrombomodulins und wenigstens einen pharmazeutisch verträglichen Träger umfasst.

2. Verwendung gemäß Anspruch 1, wobei das Thrombomodulin ein Peptid ist, das eine Aminosäuresequenz der SEQ ID Nr. 1 oder 2 umfasst.

3. Verwendung gemäß Anspruch 1, wobei das Thrombomodulin ein humanes Thrombomodulin ist.

## Revendications

1. Utilisation d'une thrombomoduline pour la production d'une composition pour le traitement d'une atteinte hépatique, dans laquelle ladite composition comprend une quantité efficace anti-atteinte hépatique d'une thrombomoduline et au moins un transporteur pharmaceutiquement acceptable.

2. Utilisation selon la revendication 1, dans laquelle ladite thrombomoduline est un peptide comprenant une séquence d'acides aminés SEQ ID No. 1 ou 2.

3. Utilisation selon la revendication 1, dans laquelle ladite thrombomoduline est une thrombomoduline humaine.
